(19) **Europäisches Patentamt**

**European Patent Office**

**Office européen des brevets**

(11) Veröffentlichungsnummer: **0 316 720 B1**

(12) # EUROPÄISCHE PATENTSCHRIFT

(45) Veröffentlichungstag der Patentschrift: **15.01.92**

(21) Anmeldenummer: **88118595.3**

(22) Anmeldetag: **09.11.88**

(51) Int. Cl.⁵: **A61M 5/14**, F16K 7/06

(54) **Klemme zum Einstellen des Durchflussquerschnittes eines Schlauches, insbesondere für medizinische Einmalgeräte.**

(30) Priorität: **17.11.87 DE 3738965**

(43) Veröffentlichungstag der Anmeldung:
**24.05.89 Patentblatt 89/21**

(45) Bekanntmachung des Hinweises auf die
Patenterteilung:
**15.01.92 Patentblatt 92/03**

(84) Benannte Vertragsstaaten:
**AT BE CH ES FR GB IT LI LU NL SE**

(56) Entgegenhaltungen:
**DE-A- 2 242 539**
**DE-A- 2 718 985**
**US-A- 4 340 201**
**US-A- 4 697 785**

(73) Patentinhaber: **Forberg, Hans-Jürgen**
**Sebenter Weg 4**
**W-2432 Damlos(DE)**

(72) Erfinder: **Forberg, Hans-Jürgen**
**Sebenter Weg 4**
**W-2432 Damlos(DE)**

(74) Vertreter: **Wilcken, Thomas, Dipl.-Ing. et al**
**Musterbahn 1**
**W-2400 Lübeck(DE)**

## Beschreibung

Die Erfindung betrifft eine Klemme zum Einstellen des Durchflußquerschnittes eines Schlauches, insbesondere für medizinische Einmalgeräte, bestehend aus einem im wesentlichen U-förmigen, länglichen Klemmgehäuse und einer darin längsverschieblichen Klemmrolle, wobei ein erster Teil des Schlauches zwischen dem Steg des Gehäuses und der Umfangsfläche der Klemmrolle einklemmbar ist, sowie aus einem zu dem Steg winklig angeordneten, sich in Gehäuselängsrichtung verengenden, weiteren Klemmbereich zum Einstellen des übrigen, den gewünschten Durchflußquerschnitt bildenden Teil des Schlauches.

Mit Hilfe einer solchen Klemme kann der Durchflußquerschnitt eines Schlauches durch Verstellen der Klemmrolle in dem Klemmgehäuse verengt und so die Durchflußmenge eines flüssigen Mediums eingestellt werden.

Eine derartige Klemme ist aus der DE-A-22 42 539 bekannt. Sie ist im Steg des Gehäuses mit einer sich in der Breite verjüngenden, kanalförmigen Aussparung versehen, die eine solche Tiefe aufweist, daß sich der flexible Schlauch unter Abwinkelung frei in die jeweilige Querschnittsstelle der Aussparung hineinzwängen kann, wodurch der entsprechende Durchflußquerschnitt eingestellt wird.

Aus der DE-A-27 18 985 ist eine weitere Klemme für einen Schlauch mit einem länglichen, U-förmigen Grundkörper bekannt, wobei der Steg des Körpers als ebene Klemmfläche ausgebildet ist und eine in dessen Seitenwänden ortsfest gelagerte, halbkreisförmige Klemmscheibe mit einem Betätigungshebel vorgesehen ist, deren Dicke dem Abstandsmaß der Seitenwände entspricht. Die Klemmscheibe hat über einen Viertelkreisumfang eine Ausnehmung konstanter radialer Tiefe, aber stetig abnehmender Breite, so daß der Schlauch eine L-förmige Verformung erfährt.

In der DE-A-20 43 551 ist wiederum eine medizinische Klemme beschrieben, die aus einem länglichen, U-förmigen Gehäuse und aus einer darin in Längsrichtung geführt einstellbaren Klemmrolle besteht, wobei der flexible Schlauch zwischen dem Steg des Gehäuses und dem Umfang der Klemmrolle eingeklemmt wird. In dem Gehäusesteg ist eine mittige, sich in der Breite verjüngende und in Gehäuselängsrichtung verlaufende Nut ausgebildet, in die ein Teil des Schlauches hineingezwängt wird, um den jeweiligen Durchflußquerschnitt zu bilden.

Bei allen genannten Ausführungen hat sich im praktischen Gebrauch herausgestellt, daß sich der jeweils eingestellte Strömungsquerschnitt des betreffenden Schlauchteiles schließlich noch weiter verkleinert, was darauf zurückgeführt wird, daß sich die als Verformungswiderstand äußernde Rückstellfähigkeit des flexiblen Schlauches wie auch dessen Wandstärke durch den sogenannten Kaltfluß aufgrund der ständigen Belastung im Einstellzustand ändert. Die Durchflußmenge durch den Schlauch wird infolgedessen ebenfalls ständig geringer und kann bei sehr geringem Durchflußquerschnitt praktisch zum Stillstand kommen, so daß jeweils eine gewisse Zeit nach der Anfangseinstellung eine Nachregulierung erforderlich und keine feste Zuordnung von Positionen der Klemmrolle und Durchflußmenge möglich ist.

Der Erfindung liegt die Aufgabe zugrunde, eine Klemme der einleitend angeführten Art dahingehend zu verbessern, daß die einmal eingestellte Durchflußmenge in sehr engen Toleranzen erhalten bleibt, so daß eine Nachregulierung nicht erforderlich ist, und bei der ein leichtes Einstellen der Durchflußmenge möglich ist.

Die Lösung der Aufgabe geht von der einleitend genannten Klemme aus und kennzeichnet sich dadurch, daß die längsverschiebliche Klemmrolle zur Ausbildung des weiteren Klemmbereiches an ihrem einen Umfangseckbereich mit einem konzentrischen Ringfalz versehen ist, dessen Ringflächen sämtlich als Klemmflächen für den übrigen Schlauchteil ausgebildet sind, daß eine der radialen Klemmfläche des Ringfalzes axial gegenüberliegende, in die Gehäuseseitenwand integrierte und den Durchflußquerschnitt in Gehäuselängsrichtung verengende Klemmleiste vorgesehen ist und daß die radiale Klemmfläche des Ringfalzes und/oder die Klemmfläche der Klemmleiste zwecks Ausbildung eines definierten dreieckigen Querschnitts des Klemmbereichs zur Klemmfläche des Gehäusesteges geneigt verlaufen.

Durch die erfindungsgemäße Lösung wird überraschender Weise erreicht, daß die einmal eingestellte Durchflußmenge in sehr engen Toleranzen erhalten bleibt, so daß ein Nachstellen der Klemmrolle entfällt. Der flexible Schlauch ist im Bereich des offenen Querschnittes der Klemme allseitig so eingefaßt, daß eine Veränderung des eingestellten Durchflußquerschnittes des Schlauches bei durch Kaltfluß bedingtem Nachsacken der Klemmrolle nicht möglich ist. Der sich einstellende Durchflußquerschnitt gleicht dem eines Dreiecks. Dabei wurde festgestellt, daß ein Zusammenhang zwischen der Gestalt der Dreiecksfläche und der Konstanz der einmal eingestellten Durchflußmenge insofern besteht, als das Nachsacken der Klemmrolle bei zu spitzer Dreiecksform eine Vergrößerung und bei zu stumpfer Dreiecksform eine Verkleinerung des Durchflußquerschnittes zur Folge hat. Um die optimale Querschnittsform zu erreichen, ist daher gemäß einem bevorzugten Merkmal vorgesehen, den genannten offenen Querschnitt der Klemme die Form eines zumindest im wesentlichen gleichschenkligen Dreiecks zu geben, wobei es sich ferner als vorteilhaft gezeigt hat, daß ein solches Dreieck am Rand des Gehäusesteges gebildet ist.

Eine weitere bevorzugte Ausgestaltung sieht vor, daß der Abstand der Seitenwände des Klemmgehäuses kleiner ist als die Breite der Klemmrolle, was bewirkt, das die Klemmrolle stets unter leichter Vorspannung der Seitenwände steht. Das hat den Vorteil, daß die seitliche Position der Klemmrolle spielfrei fixiert ist, was ebenfalls der Unveränderlichkeit des Durchflußquerschnittes des Schlauches nach Vornahme einer Einstellung auch während der Handhabung zugute kommt. Die leichte Vorspannung der Seitenwände verhindert auch, daß die Seitenwände unter der Abklemmkraft der Klemmrolle seitlich ausweichen, was einer Vergrößerung des offenen Querschnittes gleichkäme.

Die Erfindung ist nachstehend anhand eines in der anliegenden Zeichnung dargestellten Ausführungsbeispieles näher erläutert. Es zeigen:

Figur 1    eine Seitenansicht der Klemme im Längsschnitt ohne Schlauch,

Figur 2    eine Stirnansicht der Klemme gemäß dem Pfeil E in Figur 1 in der Position entsprechend dem größt möglichen Durchflußquerschnitt des geklemmten Schlauches,

Figur 3    einen Querschnitt durch die Klemme gemäß Position B in Figur 1,

Figur 4    einen Querschnitt durch die Klemme gemäß Positon C in Figur 1,

Figur 5    einen Querschnitt durch die Klemme gemäß Position D in Figur 1, das heißt mit in Ausgangsstellung befindlicher Klemmrolle, in welcher der Schlauch nicht geklemmt ist.

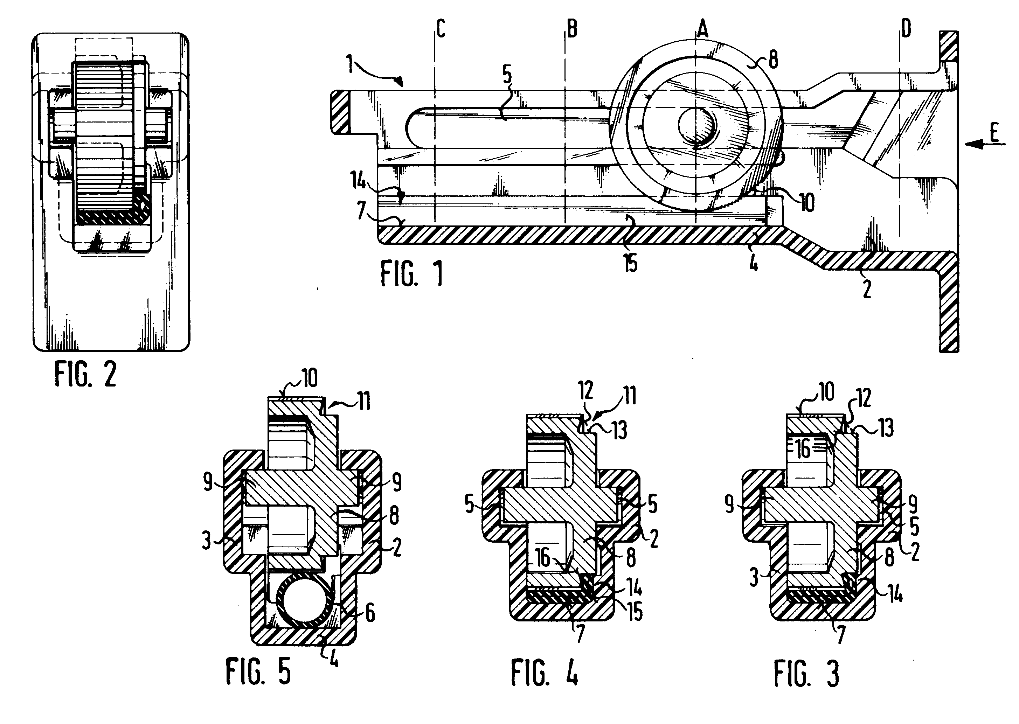

Die Vorrichtung besteht gemäß Figur 1 aus einem vorzugsweise im Spritzgießverfahren hergestellten, U-förmigen Kunststoff-Klemmgehäuse 1 mit zwei Seitenwänden 2 und 3 sowie mit einem Steg 4. In die Seitenwände 2 und 3 sind zu dem Steg 4 parallel verlaufende Führungsbahnen 5 eingearbeitet, die zu dem einen Ende des Klemmgehäuses 1 hin konisch erweitert und offen sind. Die Innenfläche 7 des Steges 4 bildet eine Klemmfläche für einen flexiblen Schlauch (6), der andererseits von einer Klemmrolle 8 eingeklemmt wird, die in ihrem Zentrum beidseitig angeordnete Achszapfen 9 aufweist, die in die Führungsbahnen 5 eingreifen. Die Umfangsfläche 10 der Klemmrolle 8 ist beispielsweise durch Rändelung griffig gestaltet und liegt der Klemmfläche 7 in einem Abstand gegenüber, der geringfügig kleiner ist als die doppelte Wandstärke des flexiblen Schlauches 6. Gemäß den Figuren 2,3 und 4 wird zwischen der Umfangsfläche 10 der Klemmrolle 8 und der Klemmfläche 7 des Gehäusesteges 4 ein erster Teil des Schlauches 6 eingeklemmt.

Die Klemmrolle 8 weist an ihrem einen Umfangseckbereich einen konzentrischen Ringfalz 11 mit einer radialen Klemmfläche 12 und mit einer Umfangsklemmfläche 13 auf. Vorzugsweise ist die radiale Klemmfläche 12 zurückspringend ausgebildet, so daß sie in Bezug auf die Klemmfläche 7 des Steges 4 geneigt verläuft. Der radialen Klemmfläche 12 des Ringfalzes 11 liegt eine Klemmleiste 14 axial gegenüber, die in die Gehäuseseitenwand 2 integriert ist und eine weitere Klemmfläche 15 bildet, die im dargestellten Fall im rechten Winkel zur Klemmfläche 7 des Gehäusesteges 4 verläuft und dabei gerundet in die Klemmfläche 7 übergeht. Des weiteren ist die Klemmleiste 14 so ausgebildet, daß ihre Klemmfläche 15 in Bezug auf die andere Seitenwand 3 des Gehäuses 1 und in Längsrichtung des letzteren konvergent verläuft und etwa gleiche Höhe aufweist wie die Klemmfläche 12, so daß bei Verschiebung der Klemmrolle 8 in Verbindung mit den Klemmflächen 12,13 des Ringfalzes 11 ein stetig kleiner werdender Querschnitt zwischen den Flächen 12,13 und 14 gegeben ist. Dieser Querschnitt hat Dreiecksform, in den der übrige Teil des Schlauches unter Bildung eines dreieckigen Durchflußquerschnittes gedrückt wird, wobei es jeweils von der axialen Stellung der Klemmrolle 8 abhängt, welche Durchströmungsrate eingestellt ist.

In alternativer Ausgestaltung ist es auch möglich, daß die Klemmfläche 15 der Klemmleiste 14 in einem stumpfen Winkel zur Klemmfläche 7 des Gehäusesteges 4 geneigt verläuft und daß die radiale Klemmfläche 12 des Ringfalzes 11 nicht zurückspringend ausgebildet ist. Zusätzlich kann diese Klemmfläche 12 aber auch zurückspringend sein, wie es die Figuren 2, 3 und 4 zeigen.

In jedem Fall ist ein Dreieck ausgebildet, durch das der betreffende Teil des Schlauches definiert dreieckförmig verformt und auch dreiseitig abgestützt wird, wenn die Klemmrolle 8 längs der Klemmleiste 14 eingestellt wird. In der Regel nimmt der betreffende Schlauchteil zumindest im wesentlichen die Form eines gleichschenkligen Dreiecks an, das in der Regel spitzwinklig und schlank ist.

Vorzugsweise ist auch der innere Eckbereich 16 des Ringfalzes 11 gerundet ausgebildet, wie es den Figuren 3, 4 und 5 entnehmbar ist, um dem Schlauch auch hier keine Ausweichmöglichkeit zu geben.Wenn es gewünscht wird, kann auch der Außenrand der radialen Klemmfläche 12 des Ringfalzes 11 abgerundet sein.

Nach einem weiteren Merkmal kann die Klemmrolle 8 mit leichtem Festsitz zwischen den Seitenwänden 3 und 4 des Gehäuses 1 angeordnet sein, damit die Klemmrolle ihre einmal eingestellte Stellung sicher beibehält und nicht nachsackt und damit die Seitenwände eine stabile Lage erhalten, so daß letztere dem Druck des eingeklemmten Schlauches nicht nachgeben. In den Figuren 2,3 und 4 ist dargestellt, wo die Klemmrolle 8 an den Seitenwänden 3 und 4 des Gehäuses 1 anliegt.

**Patentansprüche**

1. Klemme zum Einstellen des Durchflußquerschnittes eines Schlauches (6), insbesondere für medizinische Einmalgeräte, bestehend aus einem im wesentlichen im Querschnitt U-förmigen, länglichen Klemmgehäuse (1) und einer darin längsverschieblichen Klemmrolle (8), wobei ein erster Teil des Schlauches zwischen dem Steg (4) des Gehäuses und der Umfangsfläche (10) der Klemmrolle (8) einklemmbar ist, sowie aus einem zu dem Steg (4) winklig angeordneten, sich in Gehäuselängsrichtung verengenden, weiteren Klemmbereich zum Einstellen des übrigen, den gewünschten Durchflußquerschnitt bildenden Teil des Schlauches (6), dadurch gekennzeichnet, daß die längsverschiebliche Klemmrolle (8) zur Ausbildung des weiteren Klemmbereiches an ihrem einen Umfangseckbereich mit einem konzentrischen Ringfalz (11) versehen ist, dessen Ringflächen (12,13) sämtlich als Klemmflächen für den übrigen Schlauchteil ausgebildet sind, daß eine der radialen Klemmfläche (12) des Ringfalzes (11) axial gegenüberliegende, in die Gehäuseseitenwand (2) integrierte und den Durchflußquerschnitt in Gehäuselängsrichtung verengende Klemmleiste (14) vorgesehen ist und daß die radiale Klemmfläche (12) des Ringfalzes (11) und/oder die Klemmfläche (15) der Klemmleiste (14) zwecks Ausbildung eines definierten dreieckigen Querschnitts des Klemmbereichs zur Klemmfläche (7) des Gehäusesteges (4) geneigt verlaufen.

2. Klemme nach Anspruch 1, dadurch gekennzeichnet, daß die radiale Klemmfläche (12) des Ringfalzes (11) in der Höhe um die doppelte Wandstärke des Schlauches niedriger ist als die Klemmfläche (15) der Klemmleiste (14) und beide Flächen im wesentlichen die Seiten eines gedachten, annähernd gleichschenkligen, vorzugsweise spitzwinkligen Dreiecks bilden, während die umfangsmäßige Klemmfläche (13) des Ringfalzes (11) die Basisseite des gleichschenkligen Klemmdreiecks bildet.

3. Klemme nach Anspruch 1 oder 2, dadurch gekennzeichnet, daß der Bereich des Zusammentreffens der Klemmfläche (15) der Klemmleiste (14) und der Klemmfläche (7) des Gehäusesteges (4) gerundet ausgebildet ist.

4. Klemme nach Anspruch 1,2 oder 3, dadurch gekennzeichnet, daß der innere Eckbereich des Ringfalzes (11) gerundet ausgebildet ist.

5. Klemme nach wenigstens einem der Ansprüche 1 bis 4, dadurch gekennzeichnet, daß die Klemmrolle (8) mit leichtem Festsitz zwischen den Seitenwänden (2,3) des Klemmgehäuses (1) angeordnet ist.

**Claims**

1. A clamp for adjusting the through flow cross-section of a tube (6), in particular for disposable medical equipment, consisting of an elongated clamp housing (1) which is substantially U-shaped in cross-section, and of a clamping roller (8) which is longitudinally displaceable therein, in which a first part of the tube is able to be clamped between the cross-piece (4) of the housing and the peripheral area (10) of the clamping roller (8), and also consisting of a further clamping zone arranged at an angle to the cross-piece (4) and narrowing in the longitudinal direction of the housing, for adjusting the remaining part of the tube (6) forming the desired through flow cross-section, characterised in that the longitudinally displaceable clamping roller (8) for the formation of the further clamping zone is provided on its one peripheral corner region with a concentric annular groove (11), the annular surfaces (12,13) of which as a whole are constructed as clamping surfaces for the remaining part of the tube, that a clamping strip (14) is provided, lying axially opposite the radial clamping surface (12) of the annular groove (11) and which is integrated into the side wall (2) of the housing and narrows the through flow cross-section in the longitudinal direction of the housing, and that the radial clamping surface (12) of the annular groove (11) and/or the clamping surface (15) of the clamping strip (14) run in an inclined manner to the clamping surface (7) of the housing cross-piece (4) so as to form a defined triangular cross-section of the clamping zone.

2. A clamp according to Claim 1, characterised in that the radial clamping surface (12) of the annular groove (11) is lower in height by twice the wall thickness of the tube than the clamping surface (15) of the clamping strip (14) and both surfaces substantially form the sides of an imaginary, approximately isosceles, preferably acute triangle, whilst the peripheral clamping surface (13) of the annular groove (11) forms the base side of the isosceles clamping triangle.

3. A clamp according to Claim 1 or 2, characterised in that the region of meeting of the clamping surface (15) of the clamping strip (14) and of the clamping surface (7) of the housing cross-piece (4) is constructed so as to be rounded.

4. A clamp according to Claim 1, 2 or 3, characterised in that the inner corner region of the annular groove (11) is constructed so as to be rounded.

5. A clamp according to at least one of Claims 1 to 4, characterised in that the clamping roller (8) is arranged with a light close fit between the side walls (2,3) of the clamp housing (1).

**Revendications**

1. Pince de serrage destinée à régler la section transversale de passage d'un tube souple (6), en particulier pour des dispositifs médicaux utilisables une seule fois, se composant d'un boîtier (1) de pince longitudinale, sensiblement en forme de U en coupe transversale et d'un rouleau (8) de pince qui coulisse longitudinalement dans ce dernier, une première partie du tube souple pouvant être serrée entre l'entretoise (4) du boîtier et la surface périphérique (10) du rouleau (8) de pince, ainsi qu'une autre zone de serrage disposée en oblique par rapport à l'entretoise (4), se rétrécissant dans la direction longitudinale du boitier, destinée au réglage de la partie restante du tube souple (6) qui constitue la section de passage souhaitée, caractérisée en ce que le rouleau de pince (8) coulissant longitudinalement est pourvu dans sa zone périphérique, pour réaliser l'autre zone de serrage, d'un pli annulaire concentrique (11) dont les surfaces annulaires (12, 13) sont toutes réalisées sous forme de surfaces de serrage pour la partie restante du tube souple, en ce qu'il existe une nervure de serrage (14) opposée axialement à la surface radiale (12) de serrage du pli annulaire (11), intégrée dans la paroi latérale (2) de boîtier et rétrécissant la section transversale de passage dans la direction longitudinale du boîtier et en ce que la surface radiale de serrage (12) des plis annulaires (11) et/ou la surface de serrage (15) de la nervure (14) de serrage sont de tracé incliné par rapport à la surface (7) de serrage de l'entretoise ( 4) de boîtier afin de réaliser une section transversale triangulaire définie de la zone de serrage.

2. Pince de serrage selon la revendication 1, caractérisée en ce que la hauteur de la surface radiale de serrage (12) du pli annulaire (11) eut inférieure, du double de l'épaisseur de paroi du tube, à celle de la surface de serrage (15) de la nervure (14) de serrage et les deux surfaces constituent sensiblement les côtés d'un triangle imaginaire à peu près isocèle, de préférence à angle aigu, tandis que la surface de serrage périphérique (13) du pli annulaire (11) constitue le côté de base du triangle isocèle de serrage.

3. Pince de serrage selon la revendication 1 ou 2, caractérisée en ce que la zone de la rencontre de la surface de serrage (15) de la nervure (14) de serrage et la surface de serrage (7) de l'entretoise (4) de boîtier est réalisée en arrondi.

4. Pince de serrage selon la revendication 1, 2 ou 3, caractérisée en ce que la zone d'angle intérieure du pli annulaire (11) est réalisée en arrondi.

5. Pince de serrage selon l'une au moins des revendications 1 à 4, caractérisée en ce que le rouleau de pince (8) est disposé entre les parois latérales (2, 3) du boîtier (1) de pince avec un ajustement légèrement serré.

FIG. 1

FIG. 2

FIG. 3

FIG. 4

FIG. 5